# EUROPÄISCHE PATENTSCHRIFT

(11) **EP 3 820 528 B1**
(45) Veröffentlichungstag und Bekanntmachung des Hinweises auf die Patenterteilung: **30.08.2023**
(21) Anmeldenummer: 19736404.5
(22) Anmeldetag: 10.07.2019
(51) Int. Cl.: A61K 9/107, A61K 47/26, A61K 31/12, A61K 36/324, A61P 3/06, A61P 3/00, A61P 25/16, A61P 25/28, A61P 29/00, A61P 35/00, A61P 37/00, A61P 3/10, A61K 38/48, A61K 36/185, A61K 31/352, A61K 31/05, A61P 3/04

(54) **SOLUBILISAT MIT CURCUMIN UND ZUMINDEST EINEM CANNABINOID ALS WEITEREM WIRKSTOFF**
SOLUBILISATE COMPRISING CURCUMIN AND AT LEAST ONE CANNABINOID AS A FURTHER ACTIVE AGENT
SOLUBILISAT COMPRENANT DE LA CURCUMINE ET AU MOINS UN CANNABINOÏDE EN TANT QU'AUTRE PRINCIPE ACTIF

(30) Priorität: 11.07.2018 WO PCT/EP2018/068731
(43) Veröffentlichungstag der Anmeldung: 19.05.2021
(73) Patentinhaber: Aquanova AG, 64295 Darmstadt (DE)
(72) Erfinder: BEHNAM, Dariush, 64380 Rossdorf (DE)
(74) Vertreter: Tesch, Sabine
(86) Internationale Anmeldenummer: PCT/EP2019/068537
(87) Internationale Veröffentlichungsnummer: WO 2020/011854

(56) Entgegenhaltungen:
- WO-A1-2015/171445
- WO-A1-2016/022936
- WO-A1-2018/061007
- DE-U1-202012 012 130
- US-A1- 2016 081 975
- Harris Rosenkrantz ET AL: "Oral and Parenteral Formulations of Marijuana Constituents", Journal of Pharmaceutical Sciences, vol. 61, no. 7, 1 July 1972 (1972-07-01), pages 1106-1112, XP055402437, US ISSN: 0022-3549, DOI: 10.1002/jps.2600610715

## Beschreibung

Die Erfindung betrifft ein Solubilisat mit Curcumin und zumindest einem Cannabinoid. Des Weiteren betrifft die Erfindung ein Fluid enthaltend ein derartiges Solubilisat, eine Kapsel gefüllt mit einem solchen Solubilisat beziehungsweise ein Fluid und ein Nahrungsergänzungsmittel und/oder Arzneimittel enthaltend ein solches Solubilisat.

Curcumin wird als Wirkstoff basierend auf verschiedenen möglichen pharmakologischen Eigenschaften diskutiert. Beispielsweise gibt es Anhaltspunkte für die antioxidative und auch für die antiinflammatorische Wirkung des Curcumins ebenso wie für die Wirksamkeit gegen Viren und Bakterien sowie gegen Krebs. Indikationen könnten daher beispielsweise Parkinson, Alzheimer, Diabetes, kolorektale Tumoren, Bauchspeicheldrüsenkrebs und Leberfunktionsstörungen sein.

Cannabinoide sind Transformationsprodukte und synthetische Analoga einiger Terpenphenole, die hauptsächlich in Hanfpflanzen (*Cannabis*) gefunden wurden.

Der Begriff "Hanfpflanze" beziehungsweise "Cannabispflanze" umfasst den Wildtyp *Cannabis sativa* und auch Varianten davon, einschließlich *Cannabis-Chemovare,* die natürlicherweise unterschiedliche Mengen der verschiedenen Cannabinoide enthalten, *Cannabis sativa Subspecies indica* und auch Pflanzen, die das Ergebnis genetischer Kreuzungen, Selbstkreuzungen oder Hybride davon sind.

Die Cannabispflanze enthält mindestens 113 Cannabinoide aus der Gruppe der Terpenphenole. Einige dieser Cannabinoide sind Δ9-Tetrahydrocannabinol (THC), Δ8-Tetrahydrocannabinol, Cannabidiol (CBD), Cannabichromen (CBC), Cannabigerol (CBG), Cannabinol (CN), Δ9-Tetrahydrocannabivarin (THCV), Cannabicyclol (CBL), Cannabielsoin (CBE), Cannabitriol (CBT) und Cannabinodiol (CBND) .

Tetrahydrocannabinol (THC) als eine psychoaktive Substanz, sie soll hauptsächlich die berauschende Wirkung von Cannabisprodukten verursachen. In der Pflanze liegt THC natürlich in Form zweier THC-Säuren vor, welche durch Erhitzen des Pflanzenmaterials in THC umgewandelt werden.

Neben der rauschverursachenden Wirkung werden THC erhebliche medizinische Eigenschaften zugeschrieben wie schmerzlindernde und entspannende, appetitanregende, antioxidative und neuroprotektive Wirkung sowie Milderung eines Glaukoms ("grüner Star").

Cannabidiol (CBD) ist ein nicht-psychoaktives Cannabinoid, welches unter anderem ebenfalls entkrampfende, entzündungshemmende, angstlösende, immunsupprimierende, neuroprotektive und antioxidative Wirkung und eine Wirkung gegen Übelkeit hat. Zudem soll CBD hohe Blutzuckerwerte senken und den Knochenaufbau anregen können. Zusammen mit THC kann ein Synergieeffekt entstehen.

Cannabichromen (CBC) hat wie CBD keinen berauschenden Effekt. Es soll insbesondere eine antifugale, antidepressive Wirkung haben. Cannabinol (CBN) ist ein Oxidationsprodukt von THC und hat eine berauschende Wirkung. Neben schmerzlindernden, entkrampfenden und beruhigenden Effekten soll es appetitanregend und antibakteriell sowie antiinflammatorisch und antiasthmatisch wirken. Cannabigerol (CBG) ist nicht psychoaktiv und soll insbesondere eine größere schmerzlindernde Wirkung haben als THC. Tetrahydrocannabivarin (THCV) hat eine geringere psychoaktive Wirkung im Vergleich zu THC und wirkt unter anderem appetithemmend und stoffwechselanregend.

Cannabis enthält auch eine Vielzahl von Nicht-Cannabinoiden mit verschiedenen pharmakologischen Eigenschaften. Es gibt Hinweise, dass Cannabinoide wie Cannabinol (CBN), Cannabidiol (CBD) und andere die Wirkung von Δ9-THC modifizieren.

Künstliche Cannabinoide können sowohl halbsynthetisch hergestellt werden, das heißt aus natürlichen Cannabinoiden, als auch vollsynthetisch aus einfachen Grundstoffen. Auch andere Pflanzen können Wirkstoffe produzieren, die über die gleichen Mechanismen wie die Cannabinoide der Hanfpflanze im menschlichen Körper wirken.

Im Rahmen der vorliegenden Anmeldung werden mit dem Begriff "Cannabinoid" neben natürlichen und künstlichen Cannabinoiden auch Phytocannabinoide umfasst. Dazu gehören beispielsweise N-Isobutylamide aus *Echinacea* und Beta-Caryophyllen, welches in diversen Gewürzpflanzen vorkommt, sowie Yangonin aus der Kavapflanze (*Piper methysticum*) und diverse Catechine aus der Teepflanze (*Camellia sinensis*).

Um nach oraler Einnahme in den Blutkreislauf gelangen zu können, muss der Wirkstoff die Dünndarm-Blutschranke passieren, wird dann in der Leber metabolosiert und gelangt als bioverfügbarer Anteil in die Lebervene. Der Rest des insgesamt eingenommenen und im Körper freigesetzten Wirkstoffes wird entweder mikrobiell im Darm abgebaut oder mit den Fäzes beziehungsweise der Galle eliminiert.

In WO 2018/061007 A1 wird die zu verbessernde Bioverfügbarkeit von Cannabinoiden adressiert und dazu Formulierungen von Cannabinoiden beschrieben, welche wenigstens ein Öl,
wenigstens einen hydrophilen Emulgator wenigstens einen CoEmulgator und/oder Co-Solvent und wenigstens 0,1 Gew.-% eines Cannabinoids enthalten. Derartige Formulierungen können sowohl Cannabinoide als auch Curcumin enthalten.

WO 2016/022936 beschreibt eine im Hinblick auf die Magen-Darmpassage verbesserte orale Dosierungsform von Cannabinoiden oder standardisierten Marihuana-Extrakten. Die Wirkstoffbestandteile werden in einem öligen Medium mit zumindest einem Emulgator gelöst, um die Selbstemulgierung zu fördern.

Auch in der Publikation von H. Rosenkrantz et al.:"Oral and parenteral formulation of marijuana constituents" im Journal of Pharmaceutlical Sciences, Bd. 61, Nr. 7, Seiten 1106 bis 1112 werden Emulsionen beschrieben, welche THC in einem Anteil kleiner oder gleich 10 Gew.-% in einer Formulierung mit Sesamöl und Polysorbat 80 enthalten.

Der Erfinder hat bereits ein Curcuminsolubilisat geschaffen, welches eine gegenüber nativem Curcumin deutlich erhöhte Bioverfügbarkeit aufweist. Dieses Solubilisat wird in der internationalen Patentanmeldung WO 2014094921 A1 beschrieben. Überraschenderweise hat sich in mehreren Untersuchungen herausgestellt, dass dieses Curcuminsolubilisat in seiner spezifischen Formulierung aufgrund der hohen Bioverfügbarkeit auch eine unerwartet größere Wirkung auf die Verringerung von Krankheitssymptomen hat, die insbesondere mit Entzündungen oder Krebs in Verbindung stehen.

Eine Toxizität aufgrund der erfindungsgemäßen Micellierung des Wirkstoffes im Vergleich zur nativen Form konnte anhand von Untersuchungen mit MTT-Assays zur Zelllebensfähigkeit ("viability") ausgeschlossen werden. Der Nachweis der Zellvitalität mittels MTT-Test beruht dabei auf der Reduktion des gelben, wasserlöslichen Farbstoffs 3-(4,5-Dimethylthiazol-2-yl)-2,5-diphenyltetrazoliumbromid (MTT) in ein blau-violettes, wasserunlösliches Formazan.

Der Erfinder stellte sich daher die Aufgabe, eine Formulierung bereitzustellen, welche die gesundheitsfördernden bis heilenden Eigenschaften von Curcumin im Hinblick auf eine Kombination mit zumindest einem Cannabinoid als einem weiteren Wirkstoff dem menschlichen oder tierischen Organismus zugänglich macht. Insbesondere ist es eine Aufgabe der Erfindung, eine möglichst hohe Bioverfügbarkeit von Curcumin in Kombination mit zumindest einem Cannabinoid zu ermöglichen.

Aufgabe der Erfindung ist es außerdem, ernährungsphysiologisch und/oder pharmakologisch akzeptable Cannabinoide bereitzustellen. Insbesondere ist es eine Aufgabe der Erfindung, Cannabinoide in einer Form bereitzustellen, die eine für das menschliche Auge trübungsfreie Mischung mit Wasser ermöglicht.

Diese Aufgaben werden in überraschend einfacher Weise gelöst mit einem Solubilisat nach Anspruch 1. Dieses Solubilisat besteht aus Curcumin mit einem Anteil von kleiner oder gleich 10 Gew.-%, bevorzugt kleiner oder gleich 8 Gew.-%, besonders bevorzugt 3 Gew.-% bis 7 Gew.-%, vorzugsweise 1 Gew.-% bis 3 Gew.-%, und zumindest einem Cannabinoid als weiteren Wirkstoff sowie Polysorbat 80 oder einer Mischung aus Polysorbat 80 und Polysorbat 20 oder aus Polysorbat 80 und zumindest einem Zuckerester aus Speisefettsäuren (in der EU als Lebensmittelzusatzstoff E 473 zugelassen).

Die Erfindung stellt auch ein Solubilisat zur Verfügung bestehend aus zumindest einem Cannabinoid, insbesondere CBD und/oder THC, mit einem Anteil von kleiner oder gleich 10 Gew.-%, bevorzugt kleiner oder gleich 5 Gew.-%, besonders bevorzugt 0,3 Gew.-% bis 3 Gew.-%, und Polysorbat 80 oder einer Mischung aus Polysorbat 80 und Polysorbat 20 oder aus Polysorbat 80 und zumindest einem Zuckerester aus Speisefettsäuren.

Ein derartiges Solubilisat kann in Mischungen mit Curcuminsolubilisaten eingesetzt werden. Diese können auch noch weitere Wirkstoffe enthalten. Das Cannabinoid-Solibilisat selbst bietet darüber hinaus für sich genommen den Vorteil, Cannabinoide in einer Form bereitzustellen, die oral verabreicht werden kann, beispielsweise in Getränken oder als Füllung von Kapseln. Durch die erfindungsgemäße Solubilisierung des Cannabinoids, insbesondere des CBD, wird für diesen Wirkstoff ein effizienter Schutz vor Oxidation geschaffen. Gerade CBD ist ansonsten oxidationsanfällig, wodurch während der Haltbarkeitsdauer von CBD-haltigen Produkten deren Wirkstoffgehalt abnehmen kann. Dies wird mit der Erfindung verhindert. Mit dem verbesserten oxidativen Schutz des cannaboinoiden Wirkstoffes erlaubt die Erfindung, Produkte mit einer längeren Haltbarkeit bereitzustellen.

In einer vorteilhaften Weiterbildung ist das Solubilisat derart ausgeprägt, dass es oral verabreicht werden kann, insbesondere in einer Curcumin-Dosis im Bereich von 0,5 mg/kg Körpergewicht bis 1 mg/ kg Körpergewicht, bevorzugt mit einer Dosis von 0,81 mg/kg Körpergewicht, insbesondere einmal täglich.

Mit dem Begriff "Wirkstoff" wird im Rahmen dieser Anmeldung ein Stoff bezeichnet, der in pharmazeutisch wirksamer Konzentration vorliegt und vorzugsweise zum Zweck einer pharmazeutischen Wirkung beigegeben wird. Dabei sind unter der Bezeichnung des entsprechenden Wirkstoffes auch solche Stoffe zu verstehen, die im Körper in den jeweiligen Wirkstoff und/oder in seine biologisch aktive Form umgewandelt werden.

Ein Wirkstoff im Rahmen dieser Anmeldung ist ein Cannabinoid. Außerdem gehören zu den "Wirkstoffen" im Sinne dieser Anmeldung sekundäre Pflanzenstoffe, welche als chemische Verbindungen von Pflanzen weder im Energiestoffwechsel noch im aufbauenden (anabolen) oder im abbauenden (katabolen) Stoffwechsel produziert werden. Eine Gruppe der sekundären Pflanzenstoffe und damit der Wirkstoffe im Sinne dieser Anmeldung sind die Flavonoide. Auch natürliche Polyphenole wie Resveratrol oder die Polyphenole aus Süßholz und natürliche Phenole, insbesondere Chalkone wie Xanthohumol, gehören zu den "Wirkstoffen" im Sinne dieser Anmeldung. Ebenso zählen Pflanzenextrakte dazu, also Stoffe, die mit Hilfe eines Extraktionsmittels aus Pflanzen oder Pflanzenteilen herausgelöst wurden. Dazu zählen Extrakte aus Hopfen. Als "Extrakt" wird der Wirkstoff auch dann bezeichnet, wenn er noch im Extraktionsmittel gelöst vorliegt. Auch der Begriff "Auszug" ist für ein Extrakt gebräuchlich.

Zu "Wirkstoffen" im Sinne dieser Anmeldung gehören auch Enzyme. Ein Beispiel für ein Enzym als "Wirkstoff" im Sinn der vorliegenden Anmeldung ist Serrapeptase. Die Anmeldung ist jedoch nicht auf dieses Enzym beschränkt.

Das Extrakt aus dem Harz des Weihrauchbaumes, *Boswellia serrata* Extrakt, enthält mehrere pentazyklische Triterpene, welche zusammen häufig als gesamte Boswelliasäuren ("total BAs") bezeichnet werden. Mit dem Begriff "Boswelliasäuren" wird eine Gruppe chemischer Verbindungen bezeichnet, die natürlich in dem genannten Harz der Weihrauchbäume vorkommen. Die beiden Grundstrukturen sind die α-Boswelliasäure und die β-Boswelliasäure. Von den Boswelliasäuren sind auch einige Derivate bekannt, insbesondere Verbindungen, die an Position 11 eine Ketogruppe tragen und/oder an Position 3 acetyliert sind. Derzeit gelten im Hinblick auf pharmakologische Effekte insbesondere die Boswelliasäuren "αBA" α-Boswelliasäure und "ßBA" β-Boswelliasäure sowie deren Derivate "KBA" 11-keto-β-Boswelliasäure (CAS 17019-92-0) und "AKBA" 3-O-Acetyl-11-keto-β-Boswelliasäure (CAS 67416-16-9) sowie "AαBA" 3-O-Acetyl- α-Boswelliasäure und "AßBA" 3-O-Acetyl-β-Boswelliasäure als bedeutungsvoll. Besonders dem Derivat AKBA wird eine entzündungshemmende Wirkung zugeschrieben.

Im Rahmen der vorliegenden Anmeldung wird der Ausdruck "Boswellia", insbesondere in dem Begriff "Boswellia-Solubilisat" in dem Sinne gebraucht, dass sich Ausdruck "Boswellia" auf die Wirkstoffe aus dem Harz des Weihrauchbaumes bezieht, also auf zumindest eine Boswelliasäure und/oder zumindest ein Derivat einer Boswelliasäure bezieht. Der Ausdruck "Boswelliasäure-Solubilisat" bezeichnet dabei eine mizellare Formulierung zumindest einer Boswelliasäure. Diese kann auch zumindest ein Boswelliasäurederivat enthalten.

Xanthohumol ist ein natürlich in Hopfen vorkommendes Flavonoid. Dabei handelt sich um ein prenyliertes Pflanzenpolyphenol, das den Chalkonen zugeordnet wird und bisher ausschließlich im Hopfen nachgewiesen werden konnte. Dabei weisen die Bitterhopfensorten einen deutlich höheren Gehalt an Xanthohumol auf als Aromasorten. In Tests zeigte sich Xanthohumol als wirksam gegen die Entstehung und Entwicklung von Krebszellen. In Laborversuchen konnte zudem festgestellt werden, dass Xanthohumol die Nervenzellen des Gehirns schützen kann und dadurch möglicherweise helfen könnte, bei Erkrankungen wie Alzheimer oder Parkinson den Krankheitsverlauf zu verlangsamen.

Die Serratia Peptidase oder Serrapeptase ist ein proteolytisches Enzym, das durch die Bakterie Serratia, die im Darm der Seidenraupe lebt, produziert wird. Der Serrapeptase werden positive Wirkungen im Zusammenhang der Linderung von Schmerzen, Entzündungen, traumatischen Schwellungen und überschüssigen Schleimausscheidungen durch den Organismus zugeschrieben. Sie soll wie ein Entzündungshemmer und Schmerzstiller in ähnlicher Weise wie Acetylsalicylsäure, Iboprofen oder andere nicht steroidale Schmerzmittel wirken. Sie soll außerdem im Gewebe eine fibrinolytische entzündungshemmende und antiödematöse Aktivität hervorrufen. Wie alle Enzyme ist die Serrapeptase sensibel gegenüber den Säuren, die durch den Magen produziert werden. Daher ist die Bereitstellung in einer Formulierung, die eine Magenpassage ermöglicht, eine Aufgabe der Erfindung.

Das erfindungsgemäße Solubilisat kann eine oder mehrere Boswelliasäuren und/oder eine oder mehrere Boswelliasäurederivate mit einem Anteil von insgesamt kleiner oder gleich 10 Gew.-%, bevorzugt kleiner oder gleich 5 Gew.-%, besonders bevorzugt 2 Gew.-% bis 4 Gew.-% enthalten.

Aufgrund des hohen Anteils an Boswellia sieht die Erfindung in einer vorteilhaften Ausgestaltung vor, dass das Solubilisat als Quelle dass das Solubilisat als Quelle für die eine oder mehreren Boswelliasäuren und/oder eine oder mehrere Boswelliasäurederivate ein durch Extraktion mittels Essigsäure-Ethylester aus dem Harz der Pflanze *Boswellia serrata* gewonnenes Extrakt enthält, wobei Boswelliasäuren in diesem Auszug in einer Konzentration von mindestens 85 Gew.-% vorliegen.

Das erfindungsgemäße Solubilisat kann Xanthohumol mit einem Anteil von kleiner oder gleich 10 Gew.-%, bevorzugt kleiner oder gleich 5 Gew.-%, besonders bevorzugt 1 Gew.-% bis 3 Gew.-% enthalten.

Aufgrund des hohen Anteils an Xanthohumol sieht die Erfindung in einer vorteilhaften Ausgestaltung vor, dass das Solubilisat als Quelle für Xanthohumol einen ethanolischen Auszug der Hartharze aus Hopfen enthält, wobei Xanthohumol in diesem Auszug in einer Konzentration im Bereich zwischen 65 Gew.-% und 95 Gew.-%, bevorzugt auf eine Konzentration im Bereich von 80 Gew.-% bis 92 Gew.-% vorliegt. Insbesondere die unten näher erläuterten Produkte "Xantho-Flav pur" oder "Xantho-Flav" können im Rahmen der Erfindung als Xanthohumolquelle verwendet werden.

Das erfindungsgemäße Solubilisat kann Serrapeptase im Bereich bis 3 Gew.-%, bevorzugt im Bereich zwischen 0,1 Gew.-% und 2 Gew.-%, besonders bevorzugt im Bereich zwischen 0,18 Gew.-% und 0,35 Gew.-% enthalten.

Auch ein Solubilisat bestehend aus Curcumin und zumindest einem Cannabinoid als weiteren Wirkstoff kann im Rahmen der Erfindung vorteilhafterweise zur Verwendung als Arzneimittel bei der Behandlung von und/oder Vorbeugung vor mit einer Entzündung einhergehenden Krankheiten, Krebs, Alzheimer, Parkinson, Adipositas, hohen Cholesterinwerten, erhöhtem Blutzucker, Diabetes, metabolischem Syndrom und/oder

Autoimmunkrankheiten, multipler Sklerose (MS), zur Senkung von visceralem Fett, zur Thermogenese, zum Senken von Cholesterin, insbesondere LDL-Cholesterin, und/oder von Glukose im Blut und/oder von Triglyceriden im Blut, zur Verbesserung der Makulapigmentdichte, zum Vermindern von oxidativem Stress und/oder zum Vermindern der Ansammlung von Fett in den Hepatocyten, insbesondere Arzneimittel zur Behandlung von und/oder Vorbeugung vor Fettleber, Friedreich-Ataxie, lysomale Krankheiten, insbesondere Morbus Tay-Sachs, Arteriosklerose, Herzkrankheiten, Arthritis, vorgesehen sein beziehungsweise eingesetzt werden.

Insbesondere stellt die Erfindung die oben beschriebenen Solubilisate zur Verfügung zur Anwendung als entzündungshemmendes Arzneimittel und/oder als Antibiotikum und/oder als Arzneimittel mit einer Wirkung gegen Krebs, Alzheimer, Parkinson, Adipositas, hohen Cholesterinwerten, erhöhtem Blutzucker, Diabetes, metabolischem Syndrom und/oder Autoimmunkrankheiten, multipler Sklerose (MS), zur Senkung von visceralem Fett, zur Thermogenese, als cholesterinsenkendes Arzneimittel, insbesondere betreffend LDL-Cholesterin, und/oder als Arzneimittel mit einer Wirkung zur Senkung von Glukose im Blut und/oder von Triglyceriden im Blut, zur Verbesserung der Makulapigmentdichte, zum Vermindern von oxidativem Stress und/oder zum Vermindern der Ansammlung von Fett in den Hepatocyten, insbesondere Arzneimittel mit einer Wirkung gegen Fettleber, Friedreich-Ataxie, lysomale Krankheiten, insbesondere Morbus Tay-Sachs, Arteriosklerose, Herzkrankheiten, Arthritis.

Um stabile Micellen der Wirkstoffe bereitzustellen kann im Rahmen der Erfindung je nachdem, welche weiteren Komponenten im Solubilisat enthalten sind, der Emulgatoranteil, insbesondere der Polysorbatanteil, bei mindestens 70 Gew-%, bevorzugt im Bereich zwischen 75 Gew-% und 95 Gew-%, besonders bevorzugt im Bereich zwischen 79 Gew-% und 88 Gew-% liegen.

Je nach Anwendungsfall kann das erfindungsgemäße Solubilisat beispielsweise bis zu 20 Gew.-%, bevorzugt bis zu 15 Gew.-% Ethanol und/oder bis zu 25 Gew.-%, bevorzugt zwischen 12 Gew.-% und 20 Gew.-%, besonders bevorzugt bis zu 10 Gew.-% Glycerin und/oder zusätzlich bis zu 10 Gew.-%, bevorzugt bis zu 7 Gew.-% Wasser enthalten. Durch Zugabe von Ethanol kann der Anteil an Emulgator, insbesondere der Anteil von Polysorbat reduziert werden, was im Hinblick auf den von WHO empfohlenen ADI-Wert für Polysorbat (25mg/kg-Körpergewicht) einen Vorteil darstellt. Auch durch Zugabe von Glycerin kann der Anteil an Emulgator, insbesondere der Anteil von Polysorbat reduziert werden.

Die Solubilisate gemäß der Erfindung haben auch unter den physiologischen Bedingungen einer Magenpassage eine enge Partikelgrößenverteilung mit kleinen mittleren Partikelgrößen, bevorzugt reicht die Durchmesserverteilung der Micellen in einer Verdünnung des Solubilisats mit destilliertem Wasser im Verhältnis 1:500 bei pH 1,1 und 37°C von etwa d₁₀=6 nm bis etwa d₉₀=20 nm. Diese Werte wurden anhand einer Volumenverteilung ermittelt. Einzelheiten zur Partikelgrößenanalyse der Micellen der Solubilisate werden unten erläutert.

Einen Hinweis auf die im Vergleich zu nicht gemäß der Erfindung mizellierten Zusammensetzungen von zumindest einem Cannabinoid oder von Curcumin und zumindest einem Cannabinoid als weiterem Wirkstoff verbesserte Bioverfügbarkeit gibt die messtechnisch deutlich einfacher zugängliche Bestimmung der Trübung des Solubilisats. Die Trübung des Solubilisats ist vorzugsweise infolge der erfindungsgemäßen Formulierung kleiner als 25 FNU, bevorzugt kleiner als 3 FNU gemessen durch Streulichtmessung mit Infrarotlicht nach den Vorschriften der Norm ISO 7027 bei einer Verdünnung des Solubilisats im Verhältnis 1:50 in Wasser bei physiologischen Bedingungen (pH 1,1 und 37°C) .

Um die orale Anwendung eines erfindungsgemäßen Solubilisats in für den Konsumenten beziehungsweise Patienten einfacher und angenehmer Weise zu ermöglichen, stellt die Erfindung zudem eine Kapsel gefüllt mit einem oben beschriebenen Solubilisat zur Verfügung, wobei die Kapsel als Weichgelatinekapsel oder Hartgelatinekapsel oder als weiche, gelatinefreie Kapsel oder als harte, gelatinefreie Kapsel wie z.B. Cellulose-Kapsel ausgebildet ist.

Das erfindungsgemäße Solubilisat kann im Rahmen der Erfindung zudem in andere Fluide, insbesondere Flüssigkeiten eingearbeitet werden. Dabei bleiben die wirkstoffgefüllten kleinen Micellen erhalten. Somit stellt die Erfindung auch ein Fluid enthaltend oben beschriebenes Solubilisat zur Verfügung, wobei das Fluid aus der Gruppe ausgewählt ist, welche Lebensmittel, Nahrungsergänzungsmittel, Getränke, Kosmetika und pharmazeutische Produkte umfasst. Insbesondere kann das Fluid im Rahmen der Erfindung eine wässrige Verdünnung des Solubilisats umfassen.

Zur Herstellung eines erfindungsgemäßen Solubilisats mit Curcumin und zumindest einem Cannabinoid als weiteren Wirkstoff können entweder einzeln hergestellte Solubilisate miteinander gemischt werden oder ein Solubilisat enthaltend Curcumin und zumindest ein Cannabinoid als weiteren Wirkstoff wird direkt hergestellt.

Die Erfindung stellt des Weiteren ein Verfahren zum Herstellen eines oben beschriebenen Solubilisats bereit. Wird eine Co-Mizellierung von Curcumin und zumindest einem Cannabinoid als weiteren Wirkstoff angestrebt, stellt die Erfindung folgende erste Variante für ein Herstellungsverfahren bereit mit den Schritten
a) Vorlegen von Polysobat 80 oder einer Mischung aus Polysorbat 80 und Polysorbat 20 oder aus Polysorbat 80 und zumindest einem Zuckeresters aus Speisefettsäuren,
b) Zugabe des zumindest einen Cannabinoids, insbesondere in Form von CBD-Öl und/oder THC-Öl,
c) Zugabe von Curcumin Pulver

wobei in Schritt a) eine Erwärmung auf eine Temperatur im Bereich von 40°C bis 62°C, bevorzugt auf eine Temperatur im Bereich von 45°C bis 57°C, besonders bevorzugt auf eine Temperatur im Bereich von 48°C und 52°C, erfolgt
und wobei in Schritt b) eine Erwärmung auf eine Temperatur im Bereich von 82°C bis 97°C, bevorzugt auf eine Temperatur im Bereich von 83°C bis 92°C, besonders bevorzugt auf eine Temperatur im Bereich von 85°C und 89°C erfolgt, wobei diese Temperatur in Schritt c) beibehalten wird.

Durch diese Herstellungsweise wird es ermöglicht, ein Solubilisat zu produzieren, welches in wässriger Verdünnung Micellen ausbilden kann, welche sowohl mit Curcumin als auch mit zumindest einem Cannabinoid als weiteren Wirkstoff beladen sind. Dazu können die zumindest zwei Wirkstoffe auch bei einer entsprechend angepassten Temperaturführung in einem vorbereitenden Schritt miteinander gemischt und dann als Mischung gemeinsam zugegeben werden.

Im Rahmen der Erfindung wird das zumindest eine Cannabinoid insbesondere in Form von CBD-Öl, THC-Öl oder einer Mischung der beiden eingesetzt. Im Rahmen der Erfindung können als Quelle für Cannabinoide grundsätzlich alle Formulierungen verwendet werden. Beispielsweise können Öle, insbesondere auch sogenannte "Full Spektrum"-Öle, Pasten, Pulver, kristalline Formen und/oder Isolate verwendet werden, wobei alle genannten Formulierungen zumindest ein Cannabinoid enthalten. Grundsätzlich können auch Extrakte zumindest eines Cannabinoids eingesetzt werden, um ein erfindungsgemäßes Solubilisat zu erhalten. Die genannten Formulierungen können solche sein, die im Wesentlichen ein Cannabinoid enthalten, beispielweise CBD bei einem sogenannten "CBD-Öl". Es können jedoch auch Mischungen zumindest zweier Cannabinoide in einer Cannabinoidformulierung bereitgestellt werden, welche als Cannabinoid im Rahmen der Erfindung eingesetzt wird.

In Schritt b) können auch weitere Wirkstoffe wie beispielsweise *Boswellia* serrata-Extrakt und/oder Xanthohumol eingearbeitet werden.

Zusätzlich oder alternativ können in Schritt c) auch weitere Wirkstoffe wie beispielsweise Serrapeptase eingearbeitet werden. In Schritt c) kann zusätzlich oder alternativ zu weiteren Wirkstoffen eine Zugabe von MCT-Öl erfolgen.

Insbesondere kann dabei vor Schritt b) ein Schritt
b1) Zugabe von Wasser bei einer Temperatur im Bereich von 40°C bis 62°C, bevorzugt bei einer Temperatur im Bereich von 45°C bis 57°C, besonders bevorzugt bei einer Temperatur im Bereich von 48°C und 52°C, durchgeführt werden.

Zusätzlich oder alternativ kann in Schritt b1) auch die Zugabe von Ethanol bei einer Temperatur im Bereich von 40°C bis 62°C, bevorzugt bei einer Temperatur im Bereich von 45°C bis 57°C, besonders bevorzugt bei einer Temperatur im Bereich von 48°C und 52°C, durchgeführt werden.

Die Erfindung betrifft auch Solubilisate, welche sowohl allein mit Curcumin als auch allein mit jeweils einem anderen Wirkstoff beladene Micellen in wässriger Verdünnung zeigen, zumindest unmittelbar nach ihrer Herstellung. Daher stellt die Erfindung ebenso ein Verfahren zum Herstellen eines oben beschriebenen Solubilisats durch Mischen eines Curcuminsolubilisats und zumindest eines Cannabinoid-Solubilisats, insbesondere im Mengenverhältnis 1:1, zur Verfügung.

Dazu stellt die Erfindung ein Verfahren zum Herstellen eines Cannabinoid-Solubilisats zur Verfügung mit folgenden Schritten:
a) Vorlegen von Polysobat 80 oder einer Mischung aus Polysorbat 80 und Polysorbat 20 oder aus Polysorbat 80 und zumindest einem Zuckeresters aus Speiefettsäuren,
b) Zugabe des zumindest einen Cannabinoids, beispielsweise in Form von CBD-Öl und/oder THC-Öl,

wobei in Schritt a) eine Erwärmung auf eine Temperatur zumindest im Bereich von 40°C bis 62°C, bevorzugt auf eine Temperatur im Bereich von 45°C bis 57°C, besonders bevorzugt auf eine Temperatur im Bereich von 48°C und 52°C, erfolgt,
und wobei in Schritt b) eine Erwärmung auf eine Temperatur im Bereich von 82°C bis 97°C, bevorzugt auf eine Temperatur im Bereich von 83°C bis 92°C, besonders bevorzugt auf eine Temperatur im Bereich von 85°C und 89°C erfolgt.

Die Temperatur kann in Schritt a) auch auf einen Wert im Bereich von 82°C bis 97°C, bevorzugt auf eine Temperatur im Bereich von 83°C bis 92°C, besonders bevorzugt auf eine Temperatur im Bereich von 85°C und 89°C erhöht werden. Vorbereitend kann ein Schritt
a1) Mischen von Glycerin und Cannabinoid, beispielsweise in Form von CBD-Öl, zum Erzeugen einer Lösung erfolgen, wobei in Schritt a1) eine Erwärmung auf eine Temperatur im Bereich von 82°C bis 97°C, bevorzugt auf eine Temperatur im Bereich von 83°C bis 92°C, besonders bevorzugt auf eine Temperatur im Bereich von 85°C und 89°C erfolgt.

Vorbereitend können im Rahmen der Erfindung auch ein Schritt
a11) Mischen von Wasser und zumindest einem Zuckerester einer Speisefettsäure erfolgen, wobei in Schritt a11) eine Erwärmung auf eine Temperatur zumindest im Bereich von 40°C bis 62°C, bevorzugt auf eine Temperatur im Bereich von 45°C bis 57°C, besonders bevorzugt auf eine Temperatur im Bereich von 48°C und 52°C, erfolgt,
   und ein Schritt
a12) Zugabe von Polysorbat 20 und/oder Polysorbat 80
   zu der in Schritt a11) erzeugten Mischung erfolgen, wobei in Schritt a12) eine Erwärmung auf eine Temperatur im Bereich von 82°C bis 97°C, bevorzugt auf eine Temperatur im Bereich von 83°C bis 92°C, besonders bevorzugt auf eine Temperatur im Bereich von 85°C und 89°C erfolgt.

Nach Schritt a1) oder nach Schritt a11) oder nach Schritt a12) wird in Schritt b) das Cannabinoid zugegeben.

Die Erfindung wird nachfolgend anhand von Ausführungsbeispielen näher erläutert. Dabei wurden die folgenden Komponenten verwendet.

### Curcumin

Als Curcumin würde das Produkt mit dem Namen "Turmeric Oleoresin Curcumin Powder 95 %" mit dem Produktcode EP-5001 der Green Leaf Extractions Pvt Limited, Kerala, Indien, verwendet. Das Curcuminpulver trägt die CAS-Nr. 458-37-7. Es handelt sich um ein Naturprodukt, welches durch Lösungsmittelextraktion der Rhizome von *Curcuma Longa* gewonnen wird. Der Curcumin-Gehalt des Pulvers beträgt nach Herstellerangaben mindestens 95 %. Dieser Curcumin-Gehalt wird durch die ASTA-Methode 18.0 bestimmt.

In den unten beschriebenen Ausführungsbeispielen können alternativ zu dem erwähnten "oleoresin turmeric 95%"-Curcuminpulver von Greenleaf als Curcumin beispielsweise auch 95%iges Curcuminextrakt von Neelam Phyto-extracts, Mumbai, Indien oder Curcumin BCM-95-SG beziehungsweise Curcumin BCM-95-CG der eurochem GmbH, Gröbenzell, Deutschland oder Curcuma Oleoresin 95 % der Henry Lamotte OILS GmbH, Bremen, Deutschland verwendet werden.

### CBD

Als Quelle für ein Cannabinoid wurde CBD-Öl verwendet, nämlich einerseits das Produkt "CBD Drops PRM BLK 24 %" des Herstellers Pharmahemp. Des Weiteren wurde ein CBD-Isolat mit dem Markennamen Cannapure^{®} des Herstellers Arevipharma GmbH eingesetzt, welches mindestens 98 %, synthetisches Cannabidiol enthält.

### Boswellia

Mit dem Begriff "Boswellia" wird im Rahmen der vorliegenden Anmeldung insbesondere ein Extrakt aus dem Harz der Weihrauchpflanze bezeichnet. Speziell wurde ein Extrakt der Art *boswellia serrata* verwendet. Dabei handelte es sich um ein durch Extraktion mittels Essigsäure-Ethylester aus dem Harz der Pflanze mit dem botanischen Namen *boswellia serrata* gewonnenes Extrakt mit dem Produktcode "HC22519" des Herstellers Frutarom Belgium N.V., Londerzeel, Belgien. Ein Solubilisat enthaltend diesen Extrakt wird wegen dessen Gehalts an Boswelliasäuren auch als "Boswelliasäuren-Solubilisat" bezeichnet.

Neben Extrakten aus dem Harz der Weihrauchpflanze können für den Zweck der erfindungsgemäßen Solubilisate auch Boswelliasäuren und/oder Derivate der Boswelliasäuren verwendet werden. Dabei kommen insbesondere die Alpha-Boswelliasäure (CAS-Nr. 471-66-9), die Beta-Boswelliasäure (CAS-Nr. 631-69-6) sowie deren Derivate 3-O-Acetyl-Alpha-Boswelliasäure (CAS-Nr. 89913-60-0), 3-O-Acetyl-Beta-Boswelliasäure (CAS-Nr. 5968-70-7), 11-Keto-Beta-Boswelliasäure (KBA, CAS-Nr. 17019-92-0) und 3-O-Acetyl-11-Keto-Beta-Boswelliasäure (AKBA, CAS-Nr. 67416-61-9) in Frage.

### Xanthohumol

Als Xanthohumol-Quelle wurden die Produkte "Xantho-Flav" oder "Xantho-Flav pur" der Marke "Hopsteiner" der Simon H. Steiner, Hopfen, GmbH, Mainburg, Deutschland verwendet. Bei beiden handelt es sich um ein Naturprodukt, das aus Hopfen hergestellt wird. Der aktive Inhaltsstoff ist das Hopfen-Polyphenol Xanthohumol. Es handelt sich um ein gelbfarbenes Pulver mit einem Xanthohumol-Gehalt nach Herstellerangaben zwischen 65 % und 85 % in "Xantho-Flav" und von mindestens 85 % bei "Xantho-Flav pur".

Die Konzentrationen an Xanthohumol und Isoxanthohumol in "Xantho-Flav pur" werden vom Hersteller nach der UVspektrophotometrischen Analyse oder nach HPLC EBC 7.8 über externen Kalibrierstandard reines XN (370 nm) bzw. IX (290 nm) quantifiziert. "Xantho-Flav pur" enthält das prenylierte Flavonoid Xanthohumol in sehr hoher Konzentration. Für die Ausführungsbeispiele im Rahmen der vorliegenden Anmeldung wurde "Xantho-Flav pur" der Batchnummer 9432 verwendet.

### Serrapeptase

Als Serrapeptase wurde das Produkt mit der Bezeichnung Serratiopeptidase 20 000 U/mg der Shaanxi Pionieer Biotech Co. Ltd mit der Batch-Nummer PBD 20170708 verwendet. Es handelt sich um ein gräulich weißes bis hellbraunes Pulver. Die Enzymeinheit (U) ist eine heute mittlerweile durch das Katal abgelöste Einheit zur Angabe der Enzymaktivität. Da sich bei Verwendung des Katal die Zahlenwerte ändern, wird die Enzymeinheit (U) in Medizin und klinischer Chemie weiterhin genutzt. Eine Enzymeinheit U entspricht einem Mikro-Mol Substratumsatz pro Minute.

### Polysorbat 80

Als Quelle für Polysorbat 80 wurde das Material "TEGO SMO 80 V FOOD" mit dem Spezifikationscode "K04 EU-FOOD" der Evonik Nutrition & Care GmbH, Essen, Deutschland, verwendet. Das Produkt entspricht den EU-Anforderungen des Lebensmittelzusatzstoffes E 433. In den unten beschriebenen Ausführungsbeispielen können alternativ zu dem erwähnten TEGO SMO 80 V von Evonik als Polysorbat 80 auch TEGO SMO 80 V von der InCoPA Gmbh, Illertissen, Deutschland oder Crillet 4/Tween 80-LQ-(SG) von der CRODA GmbH, Nettetal, Deutschland oder Lamesorb SMO 20 sowie Kotilen-O/1 VL von Univar oder der Kolb Distributions AG, Hedingen, Schweiz verwendet werden.

### Polysorbat 20

Als Quelle für Polysorbat 20 wurde das Material "TEGO SML 20 V FOOD" mit dem Spezifikationscode "K09 EU-FOOD" der Evonik Nutrition & Care GmbH, Essen, Deutschland, verwendet. Das Produkt entspricht den EU-Anforderungen des Lebensmittelzusatzstoffes E 432. Alternativ zu dem erwähnten TEGO SML 20 von Evonik kann im Rahmen der Erfindung als Polysorbat 20 auch Crillet 1/Tween 20-LQ-(SG) von der CRODA GmbH, Nettetal, Deutschland verwendet werden.

### Zuckerester von Speisefettsäuren

Als Zuckerester von Speisefettsäuren wurde ein Sucroseester mit der Produktbezeichnung "DUB SE 15 P" des Herstellers Stéarine Dubois eingesetzt. Dieser ist in der EU als Lebensmittelzusatzstoff E 473 zugelassen.

### Ethanol

Ethanol wurde im Rahmen der vorliegenden Anmeldung von der Berkel pfälzische Spritfabrik GmbH & Co. KG bezogen. Gemäß der Spezifikation für "Neutralalkohol unvergällt" 1411U versteuert" beträgt der Gehalt an Ethanol dieses Produkts etwa 92,6 bis 95,2 Gewichts-%.

### Glycerin

Als Glycerin wurde im Rahmen der vorliegenden Anmeldung das Produkt "Glycamed 99,7 %" der Glaconchemie GmbH, Merseburg, Deutschland, verwendet. Nach Herstellerangaben liegt der Glycerin-Gehalt dieses Produkts bei mindestens 99,5 %.

### Mittelkettige Triglyceride

Mittelkettige Triglyceride, engl. medium-chain triglycerides (MCTs) sind Triglyceride, die mittelkettige Fettsäuren enthalten. Zu den mittelkettigen Fettsäuren zählen die Capronsäure, Caprylsäure, Caprinsäure und die Laurinsäure. Es handelt sich dabei um gesättigte Fettsäuren, welche natürlich in tropischen Pflanzenfetten wie Kokosfett und Palmkernöl vorkommen. Zu einem geringen Teil sind sie auch im Milchfett enthalten. In der Natur gibt es kein reines MCT-Öl, synthetisch lassen sich jedoch reine MCT-Öle gewinnen.

Die folgenden Aspekte betreffend die Verwendung von mittelkettigen Triglyceriden sind nicht erfindungsgemäß und dienen lediglich der Veranschaulichung. Als mittelkettige Triglyceride in Solubilisaten können einzelne MCTs oder eine Mischung unterschiedlicher MCTs eingesetzt werden. Mittelkettige Triglyceride wurden als MCT-Öl Delios VK koscher des Herstellers Cognis GmbH, Monheim, Deutschland, oder als MCT-Öl (70/30) Rofetan GTCC 70/30 des Herstellers DHW Deutsche Hydrierwerke Rodleben GmbH, Dessau-Roßlau, Deutschland, CAS-Nummer 73-398-61-5 eingesetzt.

Des Weiteren können mittelkettige Triglyceride in Form des Produktes ROFETAN DTCC 70/30 (Ph. Eur.) verwendet werden. Dabei handelt es sich um ein Capryl/Caprinsäuretriglycerid mit der CAS-Nummer 73398-61-5. Das Produkt entspricht der Monografie "mittelkettige Triglyceride" des zum Anmeldetag gültigen Europäischen Arzneibuches. Hersteller sind die Ecogreen Oleochemicals DHW, Deutsche Hydrierwerke GmbH, Rodleben, Deutschland.

Wird bei der Herstellung eines Solubilisats **Wasser** zugegeben, wird destilliertes Wasser verwendet.

Die Partikelgrößenanalysen der Micellen in wässrigen Verdünnungen erfindungsgemäßer Solubilisate wurden, soweit nicht anders angegeben, gemessen nach dem Prinzip der dynamischen Lichtstreuung mit Laserlicht der Wellenlänge 780 nm. Die Partikelgrößenmessungen wurden mit dem ParticleMetrix NANOFLEX Rückstreu-Teilchenanalysator durchgeführt. Das Messprinzip beruht auf der dynamischen Lichtstreuung (DLS) in einer 180° Heterodyn-Rückstreuanordnung.

Zur experimentellen Bestimmung der Trübung der erfindungsgemäßen Solubilisate werden die Trübungsmessgeräte mit einer Standardsuspension kalibriert. Die Anzeige erfolgt somit nicht in Form der gemessenen Lichtintensität, sondern als Konzentration der Kalibriersuspension. Bei der Messung einer beliebigen Suspension bedeutet also die Anzeige, dass die betreffende Flüssigkeit die gleiche Lichtstreuung verursacht wie die Standardsuspension der angezeigten Konzentration. Der international festgelegte Trübungsstandard ist Formazin. Zu den geläufigsten Einheiten gehört die Angabe "FNU", das heißt "Formazine Nephelometric Units". Dies ist die beispielsweise in der Wasseraufbereitung verwendete Einheit für die Messung bei 90° gemäss den Vorschriften der Norm ISO 7072.

Zur Herstellung eines erfindungsgemäßen Solubilisats mit den Wirkstoffen Curcumin und zumindest einem Cannabinoid als weiteren Wirkstoff können entweder einzeln hergestellte Solubilisate miteinander gemischt werden oder ein Solubilisat enthaltend Curcumin und zumindest ein Cannabinoid oder mehrere weitere Wirkstoffe wird direkt hergestellt.

### Curcuminsolubilisate

Beispielhaft wird ein 7 %iges Curcumin-Solubilisat hergestellt. Dazu werden

| | |
|---|---|
| 925 g | Polysorbat 80 |
| 75 g | Curcuminpulver 95 % (= 71,2 g Curcumin) |

verwendet. Das Polysorbat 80 wird auf 48 bis 52°C erwärmt. Unter Rühren wird das Curcumin-Pulver zum Polysorbat gegeben und dabei weiter auf eine Temperatur im Bereich von 95 bis 97°C erwärmt. Die Zugabe des Pulvers erfolgt mit einer solchen Geschwindigkeit, dass es beim Rühren gleichmäßig in den Emulgator eingezogen wird. Nach Abkühlen auf eine Temperatur unterhalb von maximal 60°C wird das Curcumin-Solubilisat abgefüllt. Dieses Solubilisat wurde für die Herstellung eines Curcumin- und Boswellia-Solubilisats verwendet.

Bei einer Verdünnung im Verhältnis 1:500 in Wasser bei einem pH-Wert von 1,1 und einer Temperatur von 37°C hat das 7 %ige Curcumin-Solubilisat eine gemittelte Trübung von 0,9 FNU.

Es sei jedoch angemerkt, dass der Curcumin-Gehalt sich weiter erhöhen lässt, ohne damit negative Folgen, beispielsweise für die Stabilität der Micellen in Kauf nehmen zu müssen.

Außerdem kann das Polysorbat 80 ganz oder teilweise durch Polysorbat 20 beziehungsweise Zuckerester aus Speisefettsäuren ersetzt werden. So können zur Herstellung eines Curcuminsolubilisats alleine mit Polysorbat 20 beispielsweise 894 g Polysorbat 80 und 106 g 95%iges Curcumin-Pulver eingesetzt werden. Das Polysorbat 20 wird auf etwa 63 °C bis etwa 67 °C erwärmt. Unter Rühren wird das Curcuminpulver langsam zum Polysorbat 80 zugegeben. Während der Zugabe des Curcuminpulvers wird weiter auf etwa 83 °C bis etwa 87 °C erwärmt. Das entstehende Solubilisat wird langsam auf unterhalb etwa 45 °C abgekühlt und ist dann bereit zur Abfüllung.

Die Herstellung dieser Varianten entspricht ansonsten der oben beschriebenen. So können bis zu etwa 11 %ige Solubilisate hergestellt werden.

### Zur Herstellung eines

**2,4 %igen CBD-Solubilisats (wasserfrei)**
werden

| | |
|---|---|
| 900 g | Polysorbat 80 und |
| 100 g | CBD-Öl (CBD Drops PRM BLK 24 %ig, = 24 g CBD) |

verwendet. Das Polysorbat 80 wird auf 48 bis 52°C erwärmt. Unter Rühren wird das CBD-Öl zum Polysorbat gegeben und dabei weiter auf eine Temperatur im Bereich von 85 bis 89°C erwärmt. Nach Abkühlen auf eine Temperatur unterhalb von maximal 60°C wird das CBD-Öl-Solubilisat abgefüllt.

Bei einer Verdünnung im Verhältnis 1:50 in Wasser bei einem pH-Wert von 1,1 und einer Temperatur von 37°C hat das wasserfreie 2,4 %ige CBD-Öl-Solubilisat eine Trübung von 2,9 FNU.

Ein erfindungsgemäßes wasserfreies CBD-Solubilisat wie insbesondere dasjenige nach obigem Beispiel ist besonders als Füllung für Kapsel geeignet.

Ein weiteres Beispiel für ein erfindungsgemäßes Cannabinoid-Solubilisat ist ein
**2,4 %iges CBD-Solubilisat,**
für welches

| | |
|---|---|
| 100 g | CBD-Öl (CBD Drops PRM BLK 24 %ig, = 24 g CBD) |
| 27 g | Zuckerester aus Speisefettsäuren |
| 54 g | Wasser |
| 22,5 g | Glycerin und |
| 796,5 g | Polysorbat 80 |

verwendet werden. Wasser und Glycerin werden gemischt und auf eine Temperatur im Bereich von 48 °C bis 52°C erwärmt. Der Zuckerester wird unter starkem Rühren eingearbeitet. Dabei wird so stark gerührt, dass sich die der Zuckerester in Wasser und Glycerin löst. Polysorbat 80 wird unter Rühren zugegeben und unter Erwärmung auf eine Temperatur von 85°C bis 89°C homogenisiert. Dabei wird so stark gerührt, dass eine gleichmäßige Verteilung des Polysorbat 80 erfolgt. Nach Abkühlung unter Rühren auf eine Temperatur unterhalb von 60°C wird das CBD-Öl unter starkem Rühren eingearbeitet und unter erneuter Erwärmung auf eine Temperatur von 85°C bis 89°C vollständig homogenisiert. Dabei wird so stark gerührt, dass eine gleichmäßige Verteilung des CBD-Öls erfolgt. Das Produkt wird auf eine Temperatur unterhalb von 60°C abgekühlt und abgefüllt. Es wird dann bei maximal 25°C dunkel gelagert.

Bei einer Verdünnung im Verhältnis 1:50 in Wasser bei einem pH-Wert von 1,1 und einer Temperatur von 37°C hat das 2,4 %ige CBD-Öl-Solubilisat eine Trübung von 3,6 FNU. Es ist besonders gut als Zusatz für Getränke geeignet.

**3 %iges CBD-Solubilisat (wasserfrei),**
Es werden

| | |
|---|---|
| 31,2 g | CBD-Isolat (Cannapure^{®} mind. 98 %ig, = 30,5 g CBD) |
| 62 g | Glycerin und |
| 906,8 g | Polysorbat 80 |

verwendet. Glycerin und CBD-Isolat werden gemischt und auf eine Temperatur im Bereich von 85°C bis 89°C erwärmt. Die Erwärmung erfolgt mit einer solchen Geschwindigkeit, dass sich das CBD vollständig in Glycerin löst und eine klare Lösung entsteht. Polysorbat 80 wird bei einer Temperatur von 85°C bis 89°C unter Rühren zugegeben. Dabei wird so stark gerührt, dass eine gleichmäßige Verteilung des Polysorbat 80 erfolgt. Das Produkt wird auf eine Temperatur unterhalb von 60°C abgekühlt und abgefüllt. Es wird dann bei maximal 25°C dunkel gelagert.

Bei einer Verdünnung im Verhältnis 1:50 in Wasser bei einem pH-Wert von 1,1 und einer Temperatur von 37°C hat das 2,4 %ige CBD-Öl-Solubilisat eine Trübung von 2,7 FNU. Es ist besonders gut als Füllung für Kapseln geeignet.

Insbesondere für Getränke kann mit einer Reduzierung der Polysorbatmenge im Rahmen der Erfindung auch folgendes
**3 %iges CBD-Solubilisat,**
hergestellt werden. Dazu werden

| | |
|---|---|
| 31,5 g | CBD-Isolat (Cannapure^{®} mind. 98 %ig, = 30,5 g CBD) |
| 27 g | Zuckerester aus Speisefettsäuren |
| 54 g | Wasser |
| 22,5 g | Glycerin und |
| 865 g | Polysorbat 80 |

verwendet. Wasser und Zuckerester werden gemischt und auf eine Temperatur im Bereich von 48 °C bis 52°C erwärmt. Polysorbat 80 wird unter Rühren zugegeben und unter Erwärmung auf eine Temperatur von 85°C bis 89°C homogenisiert. Dabei wird so stark gerührt, dass eine gleichmäßige Verteilung des Polysorbat 80 erfolgt. Es erfolgt eine Abkühlung unter Rühren auf eine Temperatur unterhalb von 60°C. Separat werden Glycerin und das CBD-Isolat gemischt und langsam auf eine Temperatur von 85°C bis 89°C vollständig homogenisiert, das heißt, bis eine klare Lösung entstanden ist. Anschließend wird das Polysorbat-Zuckerester-Gemisch unter starkem Rühren in das CBD-Glycerin-Gemisch eingearbeitet. Dabei wird unter erneutem Erwärmen auf eine Temperatur von 85°C bis 89°C so stark gerührt, dass eine gleichmäßige Verteilung erfolgt. Das Produkt wird auf eine Temperatur unterhalb von 60°C abgekühlt und abgefüllt. Es wird dann bei maximal 25°C dunkel gelagert.

Bei einer Verdünnung im Verhältnis 1:50 in Wasser bei einem pH-Wert von 1,1 und einer Temperatur von 37°C hat dieses 3 %ige CBD-Öl-Solubilisat eine Trübung von 4,1 FNU. Es ist besonders gut als Zusatz für Getränke geeignet.

Außer mit CBD-Öl können im Rahmen der Erfindung Cannabinoid-Solubilisate zusätzlich oder alternativ mit THC-Öl und/oder mit Ölen beziehungsweise Isolaten oder anderen Quellen, auch anderer Cannabinoide hergestellt und verwendet werden.

### 12 %iges Boswelliasäure-Solubilisat

Es werden

| | |
|---|---|
| 76 g | Boswellia serrata Extrakt 80 %ig (= 60,8 g Boswelliasäure) |
| 24 g | Wasser |
| 400 g | Polysorbat 20 |

verwendet.

Unter Erwärmung auf eine Temperatur im Bereich von 87 bis 93°C wird das Wasser mit dem Boswellia-Pulver gemischt. Unter Beibehaltung der Temperatur wird Polysorbat 20 eingearbeitet. Die Zugabe des Emulgators erfolgt dabei mit einer solchen Geschwindigkeit, dass sich die Fluide unter Rühren stabil zu einem Solubilisat homogenisieren. Bei der Herstellung kann eine starke Schaumbildung auftreten. Diese kann ignoriert werden, sofern bei der Abfüllung ein klares Solubilisat am Boden des Abnahmegefäßes zu erkennen ist.

Das folgende Ausführungsbeispiel eines 1,5%igen Serrapeptase-Solubilisats ist nicht erfindungsgemäß und dient lediglich der Veranschaulichung.

### 1,5%iges Serrapeptase-Solubilisat

Es werden

| | |
|---|---|
| 15 g | Serrapeptase: |
| | Serratiopeptidase 20.000 U/mg = 300 000 000 U, |
| 15 g | Wasser |
| 16,5 g | MCT Öl |
| 953,5 g | Polysorbat 80 |

verwendet.

Bei einer Temperatur im Bereich zwischen 18 und 22°C wird Wasser mit Serrapeptase gemischt und die Mischung homogenisiert. Das bedeutet, die Serrapeptase wird weitestgehend gleichmäßig im Wasser verteilt. So werden die Voraussetzungen dafür geschaffen, dass sich die Serrapeptase weitestgehend vollständig im Wasser lösen kann. Unter Erwärmung auf eine Temperatur im Bereich von 83 und 87°C wird MCT-Öl unter ständigem Rühren in die Wasser-Serrapeptase-Mischung eingearbeitet. Dabei wird so stark gerührt, dass sich die Serrapeptase gleichmäßig in Wasser löst. Bei unveränderter Temperatur wird Polysorbat 80 unter Rühren zugegeben und homogenisiert. Dabei wird so stark gerührt, dass eine gleichmäßige Verteilung des Polysorbat 80 erfolgt. Das Produkt wird auf eine Temperatur unterhalb von 60°C abgekühlt und abgefüllt. Es wird dann bei maximal 25°C dunkel gelagert.

300.000 U/g entsprechen 15 mg/g 1,5%iger Serrepaptase in enzymatischen Units. Bei einer Verdünnung in Wasser von 1:50 wurde die Trübung dieses Solubilisats unter physiologischen Bedingungen bei pH 1,1 und 37°C bestimmt. Es ergab sich ein Wert von 1,8 FNU.

### 10 %iges Xantho Flav- Solubilisat (=̂ 7,5 % Xanthohumol) mit Ethanol

Für diese Variante eines erfindungsgemäßen Xanthohumol-Solubilisats werden

| | |
|---|---|
| 100 g | Xantho Flav (=̂ 75 g Xanthohumol), |
| 150 g | Ethanol (96 %ig) Neutralalkohol Sorte 1411U |
| und | |
| 750 g | Polysorbat 80 |

verwendet.

Zunächst wird das Xantho Flav -Pulver in Ethanol gelöst und dabei auf eine Temperatur im Bereich zwischen 48 und 52°C erwärmt. Es entsteht eine homogene Lösung. Polysorbat 80 wird dann unter Erwärmen auf 83 bis 87°C zur Lösung von Xantho Flav in Ethanol zugegeben. Die Zugabe erfolgt mit einer solchen Geschwindigkeit, dass sich die beiden Fluide durch Rühren gut homogenisieren. Das entstehende Solubilisat wird auf unter 60°C abgekühlt und abgefüllt sowie dunkel und kühl, d.h. bei Temperaturen unterhalb von 25°C gelagert.

Die oben beschriebenen Solubilisate können verwendet werden, um das erfindungsgemäße Solubilisat mit Curcumin und zumindest einem weiteren Wirkstoff durch Mischen herzustellen. Dies wird anhand des Ausführungsbeispiels 1 beschrieben.

Ob eine ausreichend vollständige Homogenisierung der Komponenten zu einem erfindungsgemäßen Solubilisat abgeschlossen ist, wird bei der Herstellung aller Solubilisate im Rahmen dieser Anmeldung über Messungen der Klarheit des Produktes, welche auf die vollständige Mizellierung schließen lässt, mit Hilfe eines Laserstrahls überprüft. Eine solche Laserstrahlmessung kann beispielsweise durch Beleuchten der Probe mit Hilfe eines handelsüblichen Laserpointers, insbesondere mit einer Wellenlänge im Bereich zwischen 650 nm und 1700 nm (Spektralfarbe Rot), und anschließender Sichtkontrolle des beleuchteten beziehungsweise durchleuchteten Solubilisats. Die Kontrolle wird nicht durch Probenahme und damit außerhalb des Reaktionskessels, sondern im Reaktionskessel durchgeführt. Der Laserstrahl wird durch ein Sichtglas, welches sich auf der Vorderseite des Reaktionskessels befindet, senkrecht zum Reaktionskessel gerichtet. Wenn auf der hinteren Innenseite des Reaktionskesses vollkommen frei von Streuung nur ein Lichtpunkt erscheint, sind die entstandenen Partikelstrukturen im Reaktionskessel kleiner als die Wellenlänge des sichtbaren Lichtes und somit eine optische Bestätigung, dass der Prozess der Micellierung abgeschlossen ist.

Die folgenden Ausführungsbeispiele 1 und 2 sind in Bezug auf das Serrapeptase-Solubilisat mit MCT nicht erfindungsgemäß und dienen lediglich der Veranschaulichung.

### Ausführungsbeispiel 1

### 1,4 % Curcumin- / 2,4 % Boswelliasäure- / 1,5 % Xanthohumol- / 0,48 % CBD- / 0,3% Serrapeptase-Solubilisat

Es werden

| | |
|---|---|
| 200 g | 7%iges Curcuminsolubilisat |
| 200 g | 12%iges Bowellia-Solubilisat |
| 200 g | 7,5%iges Xanthohumol-Solubilisat und |
| 200 g | 2,4%iges wasserfreies CBD-Solubilisat |
| 200 g | 1,5%iges Serrapeptase-Solubilisat |

jeweils nach den oben beschriebenen Rezepturen verwendet.

Alle fünf Solubilisate können zum Senken der Viskosität und damit zur besseren Fließfähigkeit auf eine Temperatur im Bereich von 50°C bis 60°C erwärmt werden. Dann werden sie durch Rühren miteinander gemischt. Sobald ein homogenes Gesamtprodukt vorliegt wird dieses gegebenenfalls auf eine Temperatur unter 60°C abgekühlt und abgefüllt.

Vor der Weiterverarbeitung wie etwa die Abfüllung in Kapseln ist es zweckmäßig, das Produkt erneut aufzurühren, um es zu homogenisieren, und dazu gegebenenfalls etwas, also auf eine Temperatur von etwa 40°C bis 50°C zu erwärmen.

Bei einer Verdünnung im Verhältnis 1:50 in Wasser bei einem pH-Wert von 1,1 und einer Temperatur von 37°C hat das Solubilisat eine gemittelte Trübung von 3,4 FNU.

### Ausführungsbeispiel 2

### 1,4 % Curcumin- / 2,4 % Boswelliasäuren- / 1,5 % Xanthohumol- / 0,048 % CBD- / 0,3% Serrapeptase-Solubilisat in Direktherstellung

Es werden

| | |
|---|---|
| 15 g | Curcuminpulver 95%ig |
| 30,4 g | Boswellia Serrata Extrakt (24,3 g Boswelliasäure) |
| 20 g | Xantho Flav-Pulver (mindestens 70 % Xanthohumol = 15 g Xanthohumol) |
| 20 g | CBD-Öl (0,48 g CBD) |
| 3 g | Serrepeptase (60 000 000 U) |
| 12,6 g | Wasser |
| 20 g | Ethanol |
| 3,3 g | MCT-Öl |
| 160 g | Polysorbat 20 und |
| 715,7 g | Polysorbat 80 |

verwendet.

Polysorbat 80 und Polysorbat 20 werden bei einer Temperatur im Bereich von 48°C bis 52°C gemischt. Dabei wird so stark gerührt, dass eine homogene Mischung entsteht. Es werden Wasser und Ethanol zugegeben. Dabei wird bei einer Temperatur im Bereich von 48°C bis 52°C so stark gerührt, dass eine homogene Mischung entsteht. Langsam werden unter ständigem Rühren Xanthohumol und Boswellia, zudosiert. Anschließend wird die Temperatur unter starkem Rühren auf 63°C bis 67°C erhöht. Dann werden Curcumin, Serrapeptase sowie CBD- und MCT-Öl in die Mischung eingearbeitet. Es wird auf eine gute Durchmischung und Homogenität des Produktes geachtet. Gegebenenfalls werden Pausen zwischen der Zugabe einer Substanz und der Zugabe der nächsten Substanz eingelegt. Dabei wird derart langsam zudosiert und gerührt, dass die jeweils zuzugebende Zutat gleichmäßig in die Vorlage eingearbeitet wird.

Die Temperatur wird weiter auf einen Wert im Bereich von 85°C bis 89°C erhöht. Die Erwärmung wird unter ständigem Rühren derart langsam durchgeführt, dass die sich erwärmende Mischung homogen bleibt und ein klares Produkt entsteht. Nach Abkühlen auf eine Temperatur unterhalb von 60°C wird das Produkt abgefüllt. Es ist dunkel und viskos und wird dunkel bei Temperaturen von maximal 25°C gelagert. Vor der Weiterverarbeitung, wie etwa die Abfüllung in Kapseln, ist es zweckmäßig, das Produkt erneut aufzurühren, um es zu homogenisieren und dazu ggf. etwas, also auf eine Temperatur von etwa 40 bis 50°C, zu erwärmen.

Bei einer Verdünnung im Verhältnis 1:50 in Wasser bei einem pH-Wert von 1,1 und einer Temperatur von 37°C hat das Solubilisat eine Trübung von 3,4 FNU.

### Ausführungsbeispiel 3

### 3öCurcumin- / 3,2% Boswelliasäuren- /1,6% Xanthohumol- / 1% CBD-Öl-Solubilisat

Es werden

| | |
|---|---|
| 40,5 g | Boswellia serrata extrakt 80%ig (32,4% Boswelliasäure) |
| 31,5 g | Curcuminpulver 95%ig (29,925 g Curcumin) |
| 20,7 g | Xantho Flav Pulver mit mindestens 80% Xanthohumol (16,5 g Xanthohumol) |
| 54,3 g | Wasser |
| 45 g | Ethanol |
| 315 g | Polysorbat 20 |
| 483 g | Polysorbat 80 |
| 10 g | CBD-Öl: Cannabidiol, 30%ig, |

verwendet. Cannabidiol (CBD) ist ein kaum psychoaktives Cannabinoid aus dem weiblichen Hanf *Cannabis sativa* beziehungsweise *Cannabis indica.* Eingesetzt wurde ein nicht THC-freies CBD-Öl Das bedeutet, Spuren von THC können darin enthalten sein. Tetrahydrocannabinol (THC) ist für die berauschenden Effekte von Hanfpflanzen verantwortlich.

Unter Erwärmung auf eine Temperatur im Bereich von 48 bis 52°C werden Polysorbat 20 und Polysorbat 80 unter Rühren miteinander homogenisiert und dabei ineinander gelöst. Unter Beibehaltung der Temperatur wird die Emulgatormischung mit dem Wasser und Ethanol versetzt. Dabei wird so stark gerührt, dass das Wasser und das Ethanol gleichmäßig in der Emulgatorlösung gelöst werden. Bei unveränderter Temperatur werden das Boswellia serrata Extrakt und das Xanthohumol unter Rühren in das mit Wasser verdünnte Emulgatorgemisch eingearbeitet. Die Zugabe erfolgt mit einer derart langsamen Geschwindigkeit, dass das Boswellia serrata Extrakt und das Xanthohumol gleichmäßig in die verdünnte Emulgatorlösung unter Rühren eingezogen werden. Anschließend wir die Temperatur unter starkem Rühren auf einen Bereich zwischen 63°C bis 67°C erhöht. Das Curcuminpulver wird unter Rühren eingearbeitet. Die Temperatur wird weiter auf einen Wert im Bereich zwischen 85°C und 89°C erhöht. Dabei wird so stark gerührt, dass das Curcumin gleichmäßig in der Vorlage verteilt und homogenisiert wird. Anschließend wird das CBD-Öl in die Mischung eingeareitet. Dabei wird so stark gerührt, dass das CBD-Öl gleichmäßig in der Vorlage verteilt und homogenisiert wird.

Es erfolgt eine Abkühlung auf eine Temperatur von kleiner oder gleich 45°C. Das dunkelgelbe, viskose Präparat mit einem Solubilisat von Curcumin und Boswelliasäure und Xanthohumol und CBD-Öl wird dann abgefüllt und dunkel und kühl, d.h. bei unter 25°C gelagert. Das Solubilisat sowie die wässrige Lösung daraus sind stabil homogen und kristallklar löslich.

Es ist dem Fachmann ersichtlich, dass die Erfindung nicht auf die vorstehend beschriebenen Beispiele beschränkt ist, sondern vielmehr in vielfältiger Weise variiert werden kann. Insbesondere können die Merkmale der einzeln dargestellten Beispiele auch miteinander kombiniert oder gegeneinander ausgetauscht werden. Dies betrifft insbesondere die Emulgator- und die Wirkstoffzusammensetzung des erfindungsgemäßen Solubilisats.

## Patentansprüche

1. Solubilisat bestehend aus,
Curcumin mit einem Anteil von kleiner oder gleich 10 Gew.-%, bevorzugt kleiner oder gleich 8 Gew.-%, besonders bevorzugt 3 Gew.-% bis 7 Gew.-%, vorzugsweise 1 Gew.-% bis 3 Gew.-%,
und zumindest ein Cannabinoid als zumindest einen weiteren Wirkstoff sowie
Polysorbat 80 oder einer Mischung aus Polysorbat 80 und Polysorbat 20 oder aus Polysorbat 80 und zumindest einem Zuckerester aus Speisefettsäuren,
wobei das Solubilisat optional
bis zu 20 Gew.-%, bevorzugt bis zu 15 Gew.-% Ethanol und/oder
bis zu 25 Gew.-%, bevorzugt zwischen 12 Gew.-% und 20 Gew.-%, besonders bevorzugt bis zu 10 Gew.-% Glycerin und/oder
zusätzlich bis zu 10 Gew.-%, bevorzugt bis zu 7 Gew.-% Wasser
enthält.

2. Solubilisat nach Anspruch 1,
**dadurch gekennzeichnet, dass**
das Solubilisat als zumindest einen weiteren Wirkstoff eine Substanz oder mehrere Substanzen umfasst, welche aus der Gruppe ausgewählt ist oder sind, welche
sekundäre Pflanzenstoffe, insbesondere Flavonoide, natürliche Phenole, insbesondere Chalkone wie Xanthohumol, Pflanzenextrakte, insbesondere aus dem Harz des Weihrauchbaumes, und Enzyme, insbesondere Serrapeptase,
umfasst.

3. Solubilisat bestehend aus,
zumindest einem Cannabinoid, insbesondere Cannabidiol CBD und/oder Tetrahydrocannabinol THC, mit einem Anteil von kleiner oder gleich 10 Gew.-%, bevorzugt kleiner oder gleich 5 Gew.-%, besonders bevorzugt 0,3 Gew.-% bis 3 Gew.-%,
und Polysorbat 80 oder einer Mischung aus Polysorbat 80 und Polysorbat 20 oder aus Polysorbat 80 und zumindest einem Zuckerester aus Speisefettsäuren,
wobei das Solubilisat optional
bis zu 20 Gew.-%, bevorzugt bis zu 15 Gew.-% Ethanol und/oder
bis zu 25 Gew.-%, bevorzugt zwischen 12 Gew.-% und 20 Gew.-%, besonders bevorzugt bis zu 10 Gew.-% Glycerin und/oder
zusätzlich bis zu 10 Gew.-%, bevorzugt bis zu 7 Gew.-% Wasser
enthält.

4. Solubilisat nach einem der vorangegangenen Ansprüche
zur Verwendung bei der Behandlung von und/oder Vorbeugung vor mit einer Entzündung einhergehenden Krankheiten, Krebs, Alzheimer, Parkinson, Adipositas, hohen Cholesterinwerten, erhöhtem Blutzucker, Diabetes, metabolischem Syndrom und/oder Autoimmunkrankheiten, multipler Sklerose (MS), zur Senkung von visceralem Fett, zur Thermogenese, zum Senken von Cholesterin, insbesondere LDL-Cholesterin, und/oder von Glukose im Blut und/oder von Triglyceriden im Blut, zur Verbesserung der Makulapigmentdichte, zum Vermindern von oxidativem Stress und/oder zum Vermindern der Ansammlung von Fett in den Hepatocyten, insbesondere Arzneimittel zur Behandlung von und/oder Vorbeugung vor Fettleber, Friedreich-Ataxie, lysomale Krankheiten, insbesondere Morbus Tay-Sachs, Arteriosklerose, Herzkrankheiten, Arthritis.

5. Solubilisat nach einem der Ansprüche 1 bis 3
zur Anwendung als entzündungshemmendes Nahrungsergänzungsmittel und/oder als Arzneimittel mit einer Wirkung gegen Krebs, Alzheimer, Parkinson, Adipositas, hohen Cholesterinwerten, erhöhtem Blutzucker, Diabetes, metabolischem Syndrom und/oder Autoimmunkrankheiten, multipler Sklerose (MS), zur Senkung von visceralem Fett, zur Thermogenese, als cholesterinsekendes Arzneimittel, insbesondere betreffend LDL-Cholesterin, und/oder als Arzneimittel mit einer Wirkung zur Senkung von Glukose im Blut und/oder von Triglyceriden im Blut, zur Verbesserung der Makulapigmentdichte, zum Vermindern von oxidativem Stress und/oder zum Vermindern der Ansammlung von Fett in den Hepatocyten, insbesondere Arzneimittel mit einer Wirkung gegen Fettleber, Friedreich-Ataxie, lysomale Krankheiten, insbesondere Morbus Tay-Sachs, Arteriosklerose, Herzkrankheiten, Arthritis.

6. Solubilisat nach einem der Ansprüche 1 bis 3 oder Solubilisat zur Verwendung nach einem der Ansprüche 4 oder 5, **dadurch gekennzeichnet, dass**
der Emulgatoranteil, insbesondere der Polysorbatanteil, bei mindestens 70 Gew-%, bevorzugt im Bereich zwischen 75 Gew-% und 95 Gew-%, besonders bevorzugt im Bereich zwischen 79 Gew-% und 88 Gew-% liegt
und/oder
die Durchmesserverteilung der Micellen in einer Verdünnung des Solubilisats mit destilliertem Wasser im Verhältnis 1:500 bei physiologischen Bedingungen (pH 1,1 und 37°C) von etwa d₁₀=6 nm bis etwa d₉₀=20 nm reicht,
und/oder
die Trübung des Solubilisats kleiner als 25 FNU, bevorzugt kleiner als 5 FNU, besonders bevorzugt kleiner als 3 FNU ist gemessen durch Streulichtmessung mit Infrarotlicht nach den Vorschriften der Norm ISO 7027 bei einer Verdünnung des Solubilisats im Verhältnis 1:50 oder 1:500 in Wasser bei physiologischen Bedingungen (pH 1,1 und 37°C).

7. Kapsel gefüllt mit einem Solubilisat nach einem der Ansprüche 1 bis 3 oder 6, oder Kapsel gefüllt mit einem Solubilisat zur Verwendung nach einem der Ansprüche 4 bis 6, **dadurch gekennzeichnet, dass**
die Kapsel als Weichgelatinekapsel oder Hartgelatinekapsel oder als weiche, gelatinefreie Kapsel oder als harte, gelatinefreie Kapsel, zum Beispiel als Cellulose-Kapsel, ausgebildet ist.

8. Fluid enthaltend ein Solubilisat nach einem der Ansprüche 1 bis 3 oder 6, oder Fluid enthaltend ein Solubilisat zur Verwendung nach einem der Ansprüche 4 bis 6, **dadurch gekennzeichnet, dass**
das Fluid aus der Gruppe ausgewählt ist, welche Lebensmittel, Nahrungsergänzungsmittel, Getränke, Kosmetika und pharmazeutische Produkte umfasst,
wobei das Fluid optional eine wässrige Verdünnung des Solubilisats umfasst.

9. Verfahren zum Herstellen eines Solubilisats nach einem der Ansprüche 1 oder 2 oder 6, mit folgenden Schritten
a) Vorlegen von Polysorbat 80 oder einer Mischung aus Polysorbat 80 und Polysorbat 20 oder aus Polysorbat 80 und zumindest einem Zuckerester aus Speisefettsäuren,
b) Zugabe des zumindest eines Cannabinoids,
c) Zugabe von Curcumin Pulver
wobei
in Schritt a) eine Erwärmung auf eine Temperatur im Bereich von 40°C bis 62°C, bevorzugt auf eine Temperatur im Bereich von 45°C bis 57°C, besonders bevorzugt auf eine Temperatur im Bereich von 48°C und 52°C, erfolgt
und wobei
in Schritt b) eine Erwärmung auf eine Temperatur im Bereich von 82°C bis 97°C, bevorzugt auf eine Temperatur im Bereich von 83°C bis 92°C, besonders bevorzugt auf eine Temperatur im Bereich von 85°C und 89°C erfolgt,
wobei diese Temperatur in Schritt c) beibehalten wird,
wobei optional
vor Schritt b) ein Schritt
b1) Zugabe von Wasser bei einer Temperatur im Bereich von 40°C bis 62°C, bevorzugt bei einer Temperatur im Bereich von 45°C bis 57°C, besonders bevorzugt bei einer Temperatur im Bereich von 48°C und 52°C, durchgeführt wird
und/oder wobei optional in Schritt
b1) auch die Zugabe von Ethanol bei einer Temperatur im Bereich von 40°C bis 62°C, bevorzugt bei einer Temperatur im Bereich von 45°C bis 57°C, besonders bevorzugt bei einer Temperatur im Bereich von 48°C und 52°C, durchgeführt wird.

10. Verfahren nach Anspruch 9,
wobei in Schritt b) zumindest ein weiterer Wirkstoff, insbesondere Boswellia serrata-Extrakt und/oder Xanthohumol eingearbeitet werden.

11. Verfahren nach Anspruch 9 oder 10,
wobei in Schritt c) zumindest ein weiterer Wirkstoff, insbesondere Serrapeptase, eingearbeitet wird.

12. Verfahren zum Herstellen eines Solubilisats nach einem der Ansprüche 1 oder 2 oder 6, durch Mischen eines Curcuminsolubilisats und zumindest eines Cannabinoid-Solubilisats nach Anspruch 3, insbesondere im Mengenverhältnis 1:1 der einzelnen Solubilisate zueinander.

13. Verfahren zum Herstellen eines Cannabinoid-Solubilisats nach einem der Ansprüche 3 oder 6 mit folgenden Schritten:
a) Vorlegen von Polysorbat 80 oder einer Mischung aus Polysorbat 80 und Polysorbat 20 oder aus Polysorbat 80 und zumindest einem Zuckerester aus Speisefettsäuren,
b) Zugabe des zumindest einen Cannabinoids,
wobei in Schritt a) eine Erwärmung auf eine Temperatur zumindest im Bereich von 40°C bis 62°C, bevorzugt auf eine Temperatur im Bereich von 45°C bis 57°C, besonders bevorzugt auf eine Temperatur im Bereich von 48°C und 52°C, erfolgt
und wobei in Schritt b) eine Erwärmung auf eine Temperatur im Bereich von 82°C bis 97°C, bevorzugt auf eine Temperatur im Bereich von 83°C bis 92°C, besonders bevorzugt auf eine Temperatur im Bereich von 85°C und 89°C erfolgt.

14. Verfahren nach Anspruch 13,
wobei die Temperatur in Schritt a) auf einen Wert im Bereich von 82°C bis 97°C, bevorzugt auf eine Temperatur im Bereich von 83°C bis 92°C, besonders bevorzugt auf eine Temperatur im Bereich von 85°C und 89°C erhöht wird.

15. Verfahren nach Anspruch 13 oder 14,
wobei vor Schritt a) ein Schritt
a1) Mischen von Glycerin und Cannabinoid zum Erzeugen
einer Lösung durchgeführt wird,
wobei in Schritt a1) eine Erwärmung auf eine Temperatur im Bereich von 82°C bis 97°C, bevorzugt auf eine Temperatur im Bereich von 83°C bis 92°C, besonders bevorzugt auf eine Temperatur im Bereich von 85°C und 89°C erfolgt
wobei optional vor Schritt a) ein Schritt a11) Mischen von Wasser und zumindest einem
Zuckerester einer Speisefettsäure
durchgeführt wird, wobei in Schritt a11) eine Erwärmung auf eine Temperatur zumindest im Bereich von 40°C bis 62°C, bevorzugt auf eine Temperatur im Bereich von 45°C bis 57°C, besonders bevorzugt auf eine Temperatur im Bereich von 48°C und 52°C, erfolgt, und ein Schritt
a12) Zugabe von Polysorbat 20 und/oder Polysorbat 80
zu der in Schritt a11) erzeugten Mischung
durchgeführt wird, wobei in Schritt a12) eine Erwärmung auf eine Temperatur im Bereich von 82°C bis 97°C, bevorzugt auf eine Temperatur im Bereich von 83°C bis 92°C, besonders bevorzugt auf eine Temperatur im Bereich von 85°C und 89°C erfolgt.

## Claims

1. Solubilizate, consisting of
curcumin in a content of less than or equal to 10 wt.%, preferably less than or equal to 8 wt.%, more preferably 3 wt.% to 7 wt.%, most preferably 1 wt.% to 3 wt.%;
and at least one cannabinoid as at least one further active substance; and
polysorbate 80, or a mixture of polysorbate 80 and polysorbate 20 or of polysorbate 80 and at least one sucrose ester of edible fatty acids,
wherein the solubilizate optionally contains
up to 20 wt.%, preferably up to 15 wt.% of ethanol; and/or
up to 25 wt.%, preferably between 12 wt.% and 20 wt.%, most preferably up to 10 wt.% of glycerol; and/or
additionally up to 10 wt.%, preferably up to 7 wt.% of water.

2. The solubilizate of claim 1,
**characterised in that**
the solubilizate comprises as at least one further active substance one substance or more substances selected from the group comprising
secondary phytochemicals, in particular flavonoids, natural phenols, in particular chalcones such as xanthohumol, plant extracts, in particular from the resin of the frankincense tree, and enzymes, in particular serrapeptase.

3. Solubilizate, consisting of
at least one cannabinoid, in particular cannabidiol CBD and/or tetrahydrocannabinol THC, in a content of less than or equal to 10 wt.%, preferably less than or equal to 5 wt.%, most preferably 0.3 wt.% to 3 wt.%,
and polysorbate 80, or a mixture of polysorbate 80 and polysorbate 20 or of polysorbate 80 and at least one sucrose ester of edible fatty acids,
wherein the solubilizate optionally contains
up to 20 wt.%, preferably up to 15 wt.% of ethanol; and/or
up to 25 wt.%, preferably between 12 wt.% and 20 wt.%, most preferably up to 10 wt.% of glycerol; and/or
additionally up to 10 wt.%, preferably up to 7 wt.% of water.

4. The solubilizate as claimed in any one of the preceding claims,
for use in the treatment and/or prevention of diseases involving inflammation, cancer, Alzheimer's, Parkinson's, obesity, high cholesterol, elevated blood sugar, diabetes, metabolic syndrome, and/or autoimmune diseases, multiple sclerosis (MS), for reducing visceral fat, for thermogenesis, for lowering cholesterol, in particular LDL cholesterol, and/or glucose in the blood and/or triglycerides in the blood, for improving macular pigment density, for reducing oxidative stress and/or for reducing the accumulation of fat in the hepatocytes, in particular pharmaceutical product for treating and/or preventing fatty liver disease, Friedreich's ataxia, lysosomal diseases, in particular Tay-Sachs disease, arteriosclerosis, heart diseases, arthritis.

5. The solubilizate as claimed in any one of claims 1 to 3,
for use as an anti-inflammatory dietary supplement and/or as a pharmaceutical drug with an effect against cancer, Alzheimer's, Parkinson's, obesity, high cholesterol, elevated blood sugar, diabetes, metabolic syndrome, and/or autoimmune diseases, multiple sclerosis (MS), for lowering visceral fat, for thermogenesis, as a cholesterol-lowering pharmaceutical drug, in particular with respect to LDL cholesterol, and/or as a pharmaceutical drug with an effect for lowering glucose in the blood and/or triglycerides in the blood, for improving macular pigment density, for reducing oxidative stress and/or for reducing the accumulation of fat in the hepatocytes, in particular pharmaceutical product with an effect against fatty liver disease, Friedreich's ataxia, lysosomal diseases, in particular Tay-Sachs disease, arteriosclerosis, heart diseases, arthritis.

6. The solubilizate as claimed in any one of the claims 1 to 3 or solubilizate for use according to one of claims 4 or 5,
**characterised in that**
the emulsifier content, in particular the polysorbate content, is at least 70 wt.%, preferably in the range between 75 wt.% and 95 wt.%, most preferably in the range between 79 wt.% and 88 wt.% and/or
the diameter distribution of the micelles in a dilution of the solubilizate with distilled water in a ratio of 1:500 under physiological conditions (pH 1.1 and 37 °C) extends from about d₁₀ = 6 nm to about d₉₀ = 20 nm and/or
the turbidity of the solubilizate is less than 25 FNU, preferably less than 5 FNU, most preferably less than 3 FNU, measured by scattered light measurement using infrared light according to the specifications of the ISO 7027 standard at a dilution of the solubilizate in a ratio of 1:50 or 1:500 in water under physiological conditions (pH 1.1 and 37 °C).

7. A capsule filled with a solubilizate as claimed in any one of the claims 1 to 3 or 6, or capsule filled with a solubilizate for use according to one of claims 4 to 6, **characterised in that**
the capsule is in the form of a soft gelatin capsule or a hard gelatin capsule or a soft gelatin-free capsule or a hard gelatin-free capsule, for example a cellulose capsule.

8. A fluid, containing a solubilizate as claimed in any one of claims 1 to 3 or 6, or capsule filled with a solubilizate for use according to one of claims 4 to 6, **characterised in that**
the fluid is selected from the group comprising foods, dietary supplements, beverages, cosmetics, and pharmaceutical products wherein
the fluid optinally comprises an aqueous dilution of the solubilizate.

9. A method for producing a solubilizate according to any one of claims 1, or 2, or 6,
with the following steps
(a) providing polysorbate 80, or a mixture of polysorbate 80 and polysorbate 20 or of polysorbate 80 and at least one sucrose ester of edible fatty acids;
(b) adding the at least one cannabinoid;
(c) adding curcumin powder;
wherein
in step (a) a heating to a temperature in a range from 40 °C to 62 °C, preferably to a temperature in a range from 45 °C to 57 °C, most preferably to a temperature in a range from 48 °C to 52 °C is carried out;
and wherein in step (b) a heating to a temperature in a range from 82 °C to 97 °C, preferably to a temperature in a range from 83 °C to 92 °C, most preferably to a temperature in a range from 85 °C to 89 °C is carried out;
wherein step c) comprises maintaining this temperature;
wherein, optionally, before step (b) a step (b1) adding water at a temperature in a range from 40
°C to 62 °C, preferably at a temperature in a range from 45 °C to 57 °C, most preferably at a temperature in a range from 48 °C and to 52 °C;
is carried out,
and/or wherein, optionally, in step
(b1) also adding ethanol at a temperature in a range from 40 °C to 62 °C, preferably at a temperature in a range from 45 °C to 57 °C, most preferably at a temperature in a range from 48 °C to 52 °C,
is carried out.

10. The method of claim 9,
wherein in step (b) at least one further active substance, in particular *Boswellia serrata* extract and/or xanthohumol, is incorporated.

11. The method of claim 9 or 10,
wherein in step (c) at least one further active substance, in particular serrapeptase, is incorporated.

12. A method for producing a solubilizate according to any one of claims 1, or 2, or 6,
by mixing a curcumin solubilizate and at least a cannabinoid solubilizate according to claim 3, in particular in a quantitative ratio of 1:1 of the individual solubilizates.

13. A method for producing a cannabinoid solubilizate according to any one of claims 3 or 6, with the following steps
(a) providing polysorbate 80, or a mixture of polysorbate 80 and polysorbate 20 or of polysorbate 80 and at least one sucrose ester of edible fatty acids;
(b) adding the at least one cannabinoid;
wherein in step (a) a heating to a temperature at least
in a range from 40 °C to 62 °C, preferably to a temperature in a range from 45 °C to 57 °C, most preferably to a temperature in a range from 48 °C to 52 °C is carried out;
and wherein in step (b) a heating to a temperature in a range from 82 °C to 97 °C, preferably to a temperature in a range from 83 °C to 92 °C, most preferably to a temperature in a range from 85 °C to 89 °C, is carried out.

14. The method of claim 13,
wherein in step (a) the temperature is increased to a value in the range from 82 °C to 97 °C, preferably to a temperature in a range from 83 °C to 92 °C, most preferably to a temperature in a range from 85 °C to 89 °C.

15. The method of claim 13 or 14,
wherein before step (a) a step
(a1) mixing glycerol and cannabinoid to produce a solution
is carried out,
wherein in step (a1) a heating to a temperature in a range from 82 °C to 97 °C, preferably to a temperature in a range from 83 °C to 92 °C, most preferably to a temperature in a range from 85 °C to 89 °C is carried out
wherein optinally before step (a) a step (a11) mixing of water and at least one sucrose ester of
an edible fatty acid
is carried out, wherein in step (a11) a heating to a temperature at least in a range from 40 °C to 62 °C, preferably to a temperature in a range from 45 °C to 57 °C, most preferably to a temperature in a range from 48 °C to 52 °C is carried out;
and a step
(a12) addition of polysorbate 20 and/or polysorbate 80 to the mixture produced in step (a11) is carried out, wherein in step (a12) a heating to a temperature in a range from 82 °C to 97 °C, preferably to a temperature in a range from 83 °C to 92 °C, most preferably to a temperature in a range from 85 °C to 89 °C is carried out.

## Revendications

1. Solubilisat composé
de curcumine dans une proportion inférieure ou égale à 10 % en poids, préférablement inférieure ou égale à 8 % en poids, de manière particulièrement préférable de 3 % en poids à 7 % en poids, de préférence de 1 % en poids à 3 % en poids,
et d'au moins un cannabinoïde comme au moins un autre principe actif ainsi que
de polysorbate 80 ou d'un mélange de polysorbate 80 et de polysorbate 20 ou de polysorbate 80 et d'au moins un ester de sucre d'acides gras alimentaires,
le solubilisat contenant facultativement jusqu'à 20 % en poids, préférablement jusqu'à 15 % en poids d'éthanol et/ou
jusqu'à 25 % en poids, préférablement entre 12 % en poids et 20 % en poids, de manière particulièrement préférable jusqu'à 10 % en poids de glycérol et/ou
en outre jusqu'à 10 % en poids, préférablement jusqu'à 7 % en poids d'eau.

2. Solubilisat selon la revendication 1,
**caractérisé en ce que**
le solubilisat comprend, comme au moins un autre principe actif, une ou plusieurs substances choisies dans le groupe comprenant
des substances végétales secondaires, notamment des flavonoïdes, des phénols naturels, notamment des chalcones comme le xanthohumol, des extraits de plantes, notamment de la résine de l'arbre à encens, et des enzymes, notamment la serrapeptase.

3. Un solubilisat composé
d'au moins un cannabinoïde, en particulier le cannabidiol CBD et/ou le tétrahydrocannabinol THC, dans une proportion inférieure ou égale à 10 % en poids, préférablement inférieure ou égale à 5 % en poids, de manière particulièrement préférable de 0,3 % en poids à 3 % en poids,
et de polysorbate 80 ou d'un mélange de polysorbate 80 et de polysorbate 20 ou de polysorbate 80 et d'au moins un ester de sucre d'acides gras alimentaires,
le solubilisat contenant facultativement
jusqu'à 20 % en poids, préférablement jusqu'à 15 % en poids d'éthanol et/ou
jusqu'à 25 % en poids, préférablement entre 12 % en poids et 20 % en poids, de manière particulièrement préférable jusqu'à 10 % en poids de glycérol et/ou
en outre jusqu'à 10 % en poids, préférablement jusqu'à 7 % en poids d'eau.

4. Solubilisat selon l'une des revendications précédentes,
destiné à une utilisation dans le traitement et/ou la prévention des maladies inflammatoires, du cancer, de la maladie d'Alzheimer, de la maladie de Parkinson, de l'obésité, de l'hypercholestérolémie, de l'hyperglycémie, du diabète, du syndrome métabolique et/ou des maladies auto-immunes, de la sclérose en plaques (SEP), pour réduire la graisse viscérale, pour la thermogenèse, pour réduire le cholestérol, en particulier le cholestérol LDL, et/ou le glucose dans le sang et/ou les triglycérides dans le sang, pour améliorer la densité du pigment maculaire, pour réduire le stress oxydatif et/ou pour réduire l'accumulation de graisse dans les hépatocytes, en particulier comme médicament destiné au traitement et/ou à la prévention de la stéatose hépatique, de l'ataxie de Friedreich, des maladies lysomales, en particulier la maladie de Tay-Sachs, de l'artériosclérose, des maladies cardiaques, de l'arthrose.

5. Solubilisat selon l'une des revendications 1 à 3,
destiné à une utilisation comme complément alimentaire anti-inflammatoire et/ou comme médicament ayant un effet contre le cancer, la maladie d'Alzheimer, la maladie de Parkinson, l'obésité, l'hypercholestérolémie, l'hyperglycémie, le diabète, le syndrome métabolique et/ou les maladies auto-immunes, la sclérose en plaques (SEP), pour réduire la graisse viscérale, pour la thermogenèse, comme médicament pour réduire le cholestérol, en particulier le cholestérol LDL, et/ou comme médicament ayant un effet de réduction du glucose dans le sang et/ou des triglycérides dans le sang, un effet d'amélioration de la densité du pigment maculaire, un effet de réduction du stress oxydatif et/ou un effet de réduction de l'accumulation de graisse dans les hépatocytes, en particulier comme médicament destiné au traitement et/ou à la prévention de la stéatose hépatique, de l'ataxie de Friedreich, des maladies lysomales, en particulier la maladie de Tay-Sachs, de l'artériosclérose, des maladies cardiaques, de l'arthrose.

6. Solubilisat selon l'une des revendications 1 à 3 ou solubilisat destiné à une utilisation selon l'une des revendications 4 ou 5,
**caractérisé en ce que**
la proportion d'émulsifiant, en particulier la proportion de polysorbate, est d'au moins 70 % en poids, préférablement comprise entre 75 % en poids et 95 % en poids, de manière particulièrement préférable comprise entre 79 % en poids à 88 % en poids
et/ou
la répartition des diamètres des micelles dans une dilution du solubilisat avec de l'eau distillée dans un rapport de 1:500 dans des conditions physiologiques (pH 1,1 et 37°C) va d'environ d₁₀=6 nm à environ d₉₀=20 nm,
et/ou
la turbidité du solubilisat est inférieure à 25 FNU, préférablement inférieure à 5 FNU, de manière particulièrement préférable inférieure à 3 FNU, mesurée par diffusion de la lumière à l'aide d'une lumière infrarouge selon les prescriptions de la norme ISO 7027 pour une dilution du solubilisat dans un rapport de 1:50 ou 1:500 dans de l'eau dans des conditions physiologiques (pH 1,1 et 37°C).

7. Capsule remplie d'un solubilisat selon l'une des revendications 1 à 3 ou 6, ou capsule remplie d'un solubilisat destiné à une utilisation selon l'une des revendications 4 à 6,
**caractérisée en ce que**
la capsule est conçue comme une capsule de gélatine molle ou une capsule de gélatine dure ou comme une capsule molle, exempte de gélatine, ou comme une capsule dure, exempte de gélatine, par exemple une capsule de cellulose.

8. Fluide contenant un solubilisat selon l'une des revendications 1 à 3 ou 6, ou fluide contenant un solubilisat destiné à une utilisation selon l'une des revendications 4 à 6,
**caractérisé en ce que**
le fluide est choisi dans le groupe comprenant les produits alimentaires, les compléments alimentaires, les boissons, les produits cosmétiques et les produits pharmaceutiques,
le fluide comprenant facultativement une dilution aqueuse du solubilisat.

9. Procédé de préparation d'un solubilisat selon l'une des revendications 1 ou 2 ou 6,
comprenant les étapes suivantes :
a) présentation de polysorbate 80 ou d'un mélange de polysorbate 80 et de polysorbate 20 ou de polysorbate 80 et d'au moins un ester de sucre d'acides gras alimentaires,
b) ajout d'au moins un cannabinoïde,
c) ajout de poudre de curcumine,
à l'étape a), un chauffage à une température comprise entre 40°C et 62°C, préférablement à une température comprise entre 45°C et 57°C, de manière particulièrement préférable à une température comprise entre 48°C et 52°C, étant effectué,
et
à l'étape b), un chauffage à une température comprise entre 82°C et 97°C, préférablement à une température comprise entre 83°C et 92°C, de manière particulièrement préférable à une température comprise entre 85°C et 89°C, étant effectué,
cette température étant maintenue à l'étape c), facultativement
avant l'étape b), une étape
b1) ajout d'eau à une température comprise entre 40°C et 62°C, préférablement à une température comprise entre 45°C et 57°C, de manière particulièrement préférable à une température comprise entre 48°C et 52°C, étant effectuée
et/ou, facultativement, à l'étape
b1) également l'ajout d'éthanol à une température comprise entre 40°C et 62°C, préférablement à une température comprise entre 45°C à 57°C, de manière particulièrement préférable à une température comprise entre 48°C et 52°C, étant effectué.

10. Procédé selon la revendication 9,
au moins un autre principe actif, en particulier un extrait de Boswellia serrata et/ou du xanthohumol, étant incorporé à l'étape b).

11. Procédé selon la revendication 9 ou 10,
à l'étape c), au moins un autre principe actif, en particulier de la serrapeptase, étant incorporé.

12. Procédé de préparation d'un solubilisat selon l'une des revendications 1 ou 2 ou 6,
par mélange d'un solubilisat de curcumine et d'au moins un solubilisat de cannabinoïde selon la revendication 3, en particulier dans un rapport quantitatif de 1:1 des différents solubilisats entre eux.

13. Procédé de préparation d'un solubilisat de cannabinoïde selon l'une des revendications 3 ou 6
comprenant les étapes suivantes :
a) présentation de polysorbate 80 ou d'un mélange de polysorbate 80 et de polysorbate 20 ou de polysorbate 80 et d'au moins un ester de sucre d'acides gras alimentaires,
b) ajout dudit au moins un cannabinoïde,
à l'étape a), un chauffage à une température au moins comprise entre 40°C et 62°C, préférablement à une température comprise entre 45°C et 57°C, de manière particulièrement préférable à une température comprise entre 48°C et 52°C, étant effectué
et, à l'étape b), un chauffage à une température comprise entre 82°C et 97°C, préférablement à une température comprise entre 83°C et 92°C, de manière particulièrement préférable à une température comprise entre 85°C et 89°C, étant effectué.

14. Procédé selon la revendication 13,
la température à l'étape a) étant augmentée à une valeur comprise entre 82°C et 97°C, préférablement à une température comprise entre 83°C et 92°C, de manière particulièrement préférable à une température comprise entre 85°C et 89°C.

15. Procédé selon la revendication 13 ou 14,
avant l'étape a), une étape
al) mélange de glycérol et de cannabinoïde étant
effectuée pour produire une solution,
à l'étape a1), un chauffage à une température comprise entre 82°C et 97°C, préférablement à une température comprise entre 83°C et 92°C, de manière particulièrement préférable à une température comprise entre 85°C et 89°C, étant effectué,
facultativement avant l'étape a), une étape
all) mélange d'eau et d'au moins un ester de sucre d'un acide gras alimentaire étant effectuée, à l'étape all),
un chauffage à une température au moins comprise entre 40°C et 62°C, préférablement à une température comprise entre 45°C et 57°C, de manière particulièrement préférable à une température comprise entre 48°C et 52°C, étant effectué et une étape
a12) ajout de polysorbate 20 et/ou de polysorbate 80 au mélange produit à l'étape all) étant effectuée, à l'étape a12),
un chauffage à une température comprise entre 82°C et 97°C, préférablement à une température comprise entre 83°C et 92°C, de manière particulièrement préférable à une température comprise entre 85°C et 89°C, étant effectué.
